# EUROPÄISCHE PATENTSCHRIFT

(11) **EP 1 512 392 B1**
(45) Veröffentlichungstag und Bekanntmachung des Hinweises auf die Patenterteilung: **05.03.2014**
(21) Anmeldenummer: 04020127.9
(22) Anmeldetag: 25.08.2004
(51) Int. Cl.: A61K 8/31, A61K 8/37, A61Q 19/00

(54) **Emollient Mischung für kosmetische Formulierungen**
Emollient mixture for cosmetic formulations
Melange d'émollient dans des formulations cosmetiques

(30) Priorität: 03.09.2003 DE 10341025
(43) Veröffentlichungstag der Anmeldung: 09.03.2005
(73) Patentinhaber: Cognis IP Management GmbH, 40589 Düsseldorf (DE)
(72) Erfinder: Issberner, Ulrich, 41569 Rommerskirchen (DE); Kawa, Rolf, 40789 Monheim (DE); Mitchell, Catherine, 40223 Düsseldorf (DE); Ansmann, Achim, 40699 Erkrath (DE); Jackwerth, Bettina, 40764 Langenfeld (DE)

(56) Entgegenhaltungen:
- WO-A-97/47282
- DE-A- 4 119 890
- DE-A- 10 133 399
- US-A1- 2002 182 164

## Beschreibung

### Gebiet der Erfindung

Die Erfindung betrifft spezifische Mischungen von Ölkörpern, die in kosmetische und pharmazeutische Zubereitungen einarbeitbar sind, eine gute dermatologische Verträglichkeit aufweisen und kosmetischen Formulierungen ein besonders leichtes Hautgefühl vermitteln.

### Stand der Technik

Zur Formulierung kosmetischer Zusammensetzungen stehen dem Fachmann eine ganze Reihe verschiedenartigster Emollients zur Verfügung, zu denen u.a Silikonöle, Ester, Ether, Carbonate und Alkane gehören. Jede Verbindungsklasse weist bestimmte sensorische Charakteristika auf und häufig werden Emollient-Kombinationen mit hochspreitenden du niedrigspreitenden Ölen eingesetzt, um gezielt "sensorische Profile" auf Haut und Haaren einstellen zu können. Von besonderem Interesse sind Ölmischungen mit sogenannten synergistischen Effekten. Silikonöle, insbesondere leichtflüchtige Silikonöle, werden in kosmetischen Formulierungen häufig eingesetzt, um eine besonders leichtes Hautgefühl zu vermitteln, haben jedoch aus toxikologischer und ökologischer Sicht zahlreiche Nachteile.

Deswegen wird seit geraumer Zeit nach Ersatzstoffen für Silikonöle gesucht, insbesondere nach Emollient-Mischungen, die es erlauben Kosmetika ohne Silikonöle zu formulieren, ohne dabei auf das sensorisch leichte Profil zu verzichten. Stoffe, die sich als Silikonölersatz oder teilweiser Silikonölersatz eignen, um einen "build-up"-Effekt auf Haut und Haaren zu vermeiden, sind beispielsweise aus der WO 97/47281 bekannt. Hierfür wurde der Einsatz von Ölkörpern vorgeschlagen, die ausgewählt sind aus der Gruppe der Dialkylether, der Dialkylcyclohexane, der Guerbetalkohole, der Guerbetcarbonate, der Esteröle, der Polyolpolyhydroxystearate und/oder der Hydroxycarbonsäureester. Aus der WO 97/47282 sind kosmetische und/oder pharmazeutische Zubereitungen mit speziellen Dialkylcarbonaten und Emulgatoren bekannt, die sich durch besondere sensorische Eigenschaften auszeichnen, wobei sich die Dialkylcarbonate als gleichwertige Ersatzstoffe für Siliconöle erwiesen.

Aufgabe der vorliegenden Erfindung war es, Emollient-Mischungen zur Verfügung zu stellen, die eine verbessertes sensorisches Profil aufweisen als die Verbindungen des Standes der Technik und als Ersatzstoffe für Silikonöle eingesetzt werden können.

Überraschenderweise wurde nun gefunden, dass sich das sensorische Profil von Ölmischungen häufig nicht mit dem der Einzelverbindungen korrelieren lässt und eine Kombination verschiedener Ölkörper oder Mischungen von Ölkörpern ein wesentlich besseres sensorisches Profil aufweisen als die Einzelverbindungen.

### Beschreibung der Erfindung

Gegenstand der Erfindung sind daher bei 20°C und Normaldruck flüssige, wasserfreie Zusammensetzungen auf Ölbasis, enthaltend (a) 2 - 90 Gew.-% wenigstens eines linearen und/oder vezweigten Dialkylcarbonats und (b) 2 - 95 Gew.-% wenigstens eines linearen und/oder verzweigten gesättigten aber ungesägttigten Alkans mit 8 bis 40 Kohlenstoffatomen. Erfindungsgemäß bevorzugt sind Zusammensetzungen, die (a) wenigstens 10 Gew.-% eines linearen und/oder verzweigten Dialkylcarbonats und (b) wenigstens 50 Gew.-% eines linearen und/oder verzweigten gesättigten aber ungesättigten Alkans mit 8 bis 40 Kohlenstoffatomen enthalten. Besonders bevorzugt sind Zusammensetzungen, die (a) 30 - 70 Gew.-% Dialkylcarbonat(e) und (b) 50 - 90 Gew.-% C8-C40 Alkan(e) enthalten und insbesondere Zusammensetzungen enthaltend (a) 30 - 50 Gew.-% Dialkylcarbonat(e) und (b) 50 - 90 Gew.-% C8-C40 Alkan(e).

Unter wasserfrei im Sinne der Erfindung werden Zusammensetzungen verstanden, die weniger als 10 Gew.-% Wasser, vorzugsweise weniger als 5 Gew.-% Wasser und besonders bevorzugt weniger als 3 Gew.-% Wasser enthalten. In einer besonders bevorzugten Ausführungsform enthalten die Zusammensetzungen nur rohstoffbedingte Restwassermengen.

Die erfindungsgemäßen Zusammensetzungen besteht im wesentlichen nur aus den Komponenten (a) und (b), abgesehen von rohstoffbedingten Verunreinigungen und rohstoffbedingten Restwassermengen.

Erfindungsgemäß bevorzugte Zusammensetzungen sind daher dadurch gekennzeichnet, dass sich die Mengen von (a) und (b), ggf. mit rohstoffbedingtem Restwasser (c) und ggf. mit rohstoffbedingten Verunreinigungen (d) zu 100 Gew.-% ergänzen.

Die erfindungsgemäßen Zusammensetzungen weisen üblicherweise eine Viskosität von 1 bis 20 mPa.s bei 20°C auf (Höppler Viskosimeter; Kugel 6).

Die erfindungsgemäßen Mischungen eignen sich aufgrund ihres sensorischen Profils besonders gut als Grundkörper in kosmetischen und pharmazeutischen Zusammensetzungen. Sie sind leicht verteilbar, ziehen gut auf die Haut auf, hinterlassen ein relativ geringes öliges oder fettendes, sondern vielmehr samtiges Hautgefühl. Sie sind daher auch gut als Ersatzstoff für Silikonöle geeignet.

Dialkylcarbonate und deren Herstellung sind aus dem Stand der Technik bekannt. Die Dialkylcarbonate können symmetrisch oder unsymmetrisch, verzweigt oder unverzweigt, gesättigt oder ungesättigt sein. Erfindungsgemäß können Reinsubstanzen oder Gemische verschiedener Dialkylcarbonate eingesetzt werden. Bevorzugt sind Dialkylcarbonate mit Alkylketten, die 6 bis 24 Kohlenstoffatome aufweisen. Besonders bevorzugt sind bei 20 °C und Normaldruck flüssige, lineare oder verzweigte, gesättigte Dialkylcarbonate. Erfindungsgemäß ganz besonders bevorzugt sind Di-n-octylcarbonat oder Di-(2-ethylhexyl)carbonat oder ein Gemisch dieser Substanzen. Unter diesen ist das Di-n-Octylcarbonat. bevorzugt.

Die Verbindungen lassen sich durch Umesterung von Dimethyl- oder Diethylcarbonat mit den entsprechenden Hydroxyverbindungen nach Verfahren des Standes der Technik herstellen; eine Übersicht hierzu findet sich in Chem.Rev. 96, 951 (1996**).** Typische Beispiele für Dialkyl(en)carbonate sind Umesterungsprodukte von Dimethyl- und/oder Diethylcarbonat mit Capronalkohol, Caprylalkohol, 2-Ethylhexylalkohol, Caprinalkohol, Laurylalkohol, Myristylalkohol, Cetylalkohol, Palmoleylalkohol, Stearylalkohol, Isostearylalkohol, Elaidylalkohol, Petroselinylalkohol, Linolylalkohol, Linolenylalkohol, Oleylalkohol, Rizinolalkohol, Elaeostearylalkohol, Arachidylalkohol, Gadoleylalkohol, Behenylalkohol, Erucylalkohol und Brassidylalkohol, Guerbetalkoholen, sowie deren technische Mischungen, die z.B. bei der Hochdruckhydrierung von technischen Methylestern auf Basis von Fetten und Ölen anfallen.

Die erfindungsgemäß einsetzbaren Alkane weisen eine Kettenlänge von 8 bis 40 C-Atomen aus. Sie können verzweigt oder unverzweigt sein, gesättigt oder ungesättigt. Erfindungsgemäß bevorzugt sind C8-C40-Alkane, die bei 20 °C und Normaldruck flüssig sind. Unter diesen sind verzweigte, gesättigte C8-C40-Alkane bevorzugt. Es können sowohl Reinsubstanzen eingesetzt werden als auch Substanzgemische. Üblicherweise handelt es sich um Substanzgemische verschiedener isomerer Verbindungen. Zusammensetzung die Alkane mit 10 bis 20 Kohlenstoffatome aufweisen, sind besonders bevorzugt. Eine bevorzugte Ausführungsform der Zusammensetzung ist dadurch gekennzeichnet, dass das Alkan (b) ein Gemisch aus Alkanen ist, welches wenigstens 10 Gew.-% verzweigte Alkane bezogen auf die Gesamtmenge der Alkane enthält. Vorzugsweise handelt es sich um verzweigte, gesättigte Alkane. Eine weitere bevorzugte Ausführungsform ist dadurch gekennzeichnet, dass das Alkan (b) eine Gemisch aus Alkanen ist, welches mehr als 1 Gew.-% 5,8-Diethyldodecan und/oder mehr als 1 Gew.-% Didecen enthält.
Erfindungsgemäß einsetzbar sind auch cyclische Alkane, wie z.B. naphthenische Kohlenwasserstoffe. Allerdings sind bevorzugte Ausführungsformen der Erfindung dadurch gekennzeichnet, dass das Alkan (b) ein nicht-cyclisches Alkan oder ein Gemisch aus nicht-cyclischen Alkanen ist.

Vorzugsweise enthalten die erfindungsgemäßen Zusammensetzungen keine zusätzlichen Öle oder Wachse außer die unter (a) und (b) genannten.

Eine Zusammensetzung, die (a) 2 - 90 Gew.-% Di-n-octylcarbonat und (b) 2 - 95 Gew.-% eines Diethyldodecans oder eines Didecens oder eines beliebigen Isomerengemisches dieser Substanzen enthält, ist erfindungsgemäß bevorzugt geeignet. Bezüglich der in Tabelle 1 genannten Bewertungskriterien schneiden Mischungen aus 30 Gew.-% Di-n-octylcarbonat und 70 Gew.-% Diethyldodecane, 10 Gew.-% Di-n-octylcarbonat und 90 Gew.-% Diethyldodecane sowie eine Mischung aus 50 Gew.-% Di-n-octylcarbonat und 50 Gew.-% Didecen am besten ab.

Ein weiterer Gegenstand der Erfindung ist die Verwendung der erfindungsgemäßen Zusammensetzungen als Silikonersatz in kosmetischen und pharmazeutischen Mitteln.

### Kosmetische/pharmazeutische Zubereitungen

Die erfindungsgemäßen Zusammensetzungen werden in kosmetischen und pharmazeutischen Mitteln eingesetzt, um diesen ein sensorisch sehr leichtes Hautgefühl zu vermitteln. Hierbei handelt es sich z.B. um Körperpflege-Produkte, die als Creme, Milch, Lotion oder sprühbare Emulsion formuliert werden, um Produkte zur Eliminierung des Körpergeruchs, etc. Die erfindungsgemäße Zusammensetzung lässt sich auch in tensidhaltigen Formulierungen wie z. B. Schaum- und Duschbädern, Haarshampoos und Pflegespülungen einsetzen. Ein weiterer Gegenstand der Anmeldung sind daher kosmetische Mittel, welche diese erfindungsgemäße Zusammensetzung gemäß einem der Ansprüche 1 bis 10 enthalten. Vorzugsweise ist die Zusammensetzung in einer Menge von 0,1 - 50 Gew.-% bezogen auf die Gesamtzusammensetzung enthalten und das Mittel frei von Silikonölen, insbesondere frei von Cyclomethicone.

Die kosmetischen Mittel können in Form von Emulsionen oder Dispersionen vorliegen, die Wasser und Ölphase nebeneinander enthalten. Bevorzugte kosmetische Zusammensetzungen sind solche in Form einer W/O- oder O/W-Emulsion mit den üblichen, dem Fachmann geläufigen, Konzentrationen an Ölen/Fetten/Wachsen, Emulgatoren, Wasser und den in der Kosmetik üblichen, weiteren Hilfs- und Zusatzstoffen.

Je nach Applikationszweck enthalten die kosmetischen Formulierungen eine Reihe weiterer Hilfs- und Zusatzstoffe wie beispielsweise oberflächenaktive Substanzen (Tenside, Emulgatoren), weitere Ölkörper, Perlglanzwachse, Konsistenzgeber, Verdickungsmittel, Überfettungsmittel, Stabilisatoren, Polymere, Fette, Wachse, Lecithine, Phospholipide, biogene Wirkstoffe, UV-Licht-schutzfaktoren, Antioxidantien, Deodorantien, Antitranspirantien, Antischuppenmittel, Filmbildner, Quellmittel, Insektenrepellentien, Selbstbräuner, Tyrosinaseinhibitoren (Depigmentierungsmittel), Hydrotrope, Solubilisatoren, Konservierungsmittel, Parfümöle, Farbstoffe etc., die nachstehend exemplarisch aufgelistet sind.

Die Mengen der jeweiligen Zusätze richten sich nach der beabsichtigten Verwendung.

### Oberflächenaktive Substanzen

Als oberflächenaktive Stoffe können anionische, nichtionische, kationische und/oder amphotere bzw. zwitterionische Tenside bzw. Emulgatoren oder ein beliebiges Gemisch dieser Tenside/Emulgatoren enthalten sein. Der Gehalt oberflächenaktiver Substanzen hängt von der Art der Formulierung ab, übersteigt aber üblicherweise 20 Gew.-% nicht. In tensidhaltigen kosmetischen Zubereitungen, wie beispielsweise Duschgelen, Schaumbädern, Shampoos etc. ist vorzugsweise wenigstens ein anionisches Tensid enthalten, in Cremes und Lotionen für die Körperpflege sind vorzugsweise nichtionische Tenside/Emulgatoren enthalten.

Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Monound Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Polyglycerinester, Alkylphenolpolyglycolether, Fettsäurepolyglycolester, Fettsäureamid-polyglycolether, Fettaminpolyglycolether, alkoxylierte Triglyceride, Mischether bzw. Mischformale, Alk(en)yloligoglykoside bzw. Glucoronsäurederivate - geg ebenenfalls partiell oxidiert, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für **kationische Tenside** sind quartäre Ammoniumverbindungen, wie beispielsweise das Dimethyldistearylammoniumchlorid, und Esterquats, insbesondere quatemierte Fettsäuretrialkanolaminestersalze. Typische Beispiele für **amphotere bzw. zwitterionische Tenside** sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Hinsichtlich Struktur und Herstellung dieser Stoffe sei auf einschlägige Übersichtsarbeiten auf diesem Gebiet verwiesen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Körperpflegemittel, wie Cremes, Lotionen und Milchen, enthalten üblicherweise eine Reihe weiterer Ölkörper und Emollients, die dazu beitragen, die sensorischen Eigenschaften weiter zu optimieren. Die Ölkörper sind üblicherweise in einer Gesamtmenge von 1 - 50 Gew.-%, vorzugsweise 5 - 25 Gew.-% und inbesondere 5 - 15 Gew.-% enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z. B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z. B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z. B. Finsolv® TN), lineare oder verzweigte, symmetrische oder unsymmetrische Dialkylether mit 6 bis 22 Kohlenstoffatomen pro Alkylgruppe, wie z. B. Di-n-octyl Ether (Cetiol® OE) oder Ringöffnungsprodukte von epoxidierten Fettsäureestem mit Polyolen.

### Fette und Wachse

Fette und Wachse werden den Körperpflegeprodukten als Pflegestoffe zugesetzt und auch, um die Konsistenz der Kosmetika zu erhöhen. Typische Beispiele für Fette sind Glyceride, d. h. feste oder flüssige pflanzliche oder tierische Produkte, die im Wesentlichen aus gemischten Glycerinestern höherer Fettsäuren bestehen. Auch Fettsäurepartialglyceride, d. h. technische Mono- und/oder Diester des Glycerins mit Fettsäuren mit 12 bis 18 Kohlenstoffatomen wie etwa Glycerinmono/dilaurat, -palmitat oder -stearat kommen hierfür in Frage. Als Wachse kommen u. a. natürliche Wachse, wie z. B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage.

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Verdickungsmittel

Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, Polyacrylate, (z. B. Carbopole® und Pemulen-Typen von Goodrich; Synthalene® von Sigma; Keltrol-Typen von Kelco; Sepigel-Typen von Seppic; Salcare-Typen von Allied Colloids und Cosmedia® SP und SPL von Cognis), Polyacrylamide, Polymere, Polyvinylalkohol, Polyvinylpyrrolidon und Bentonite wie z. B. Bentone® Gel VS-5PC (Rheox). Weiter in Frage kommen Elektrolyte wie Kochsalz und Ammoniumchlorid.

### Stabilisatoren

Als Stabilisatoren können Metallsalze von Fettsäuren, wie z. B. Magnesium-, Aluminium- und/oder Zinkstearat bzw. -ricinoleat eingesetzt werden.

### UV.Lichtschutzfilter und Antioxidantien

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z. B. Wärme wieder abzugeben. UV-B-Filter können öllöslich oder wasserlöslich sein. Als typische UV-A-Filter kommen insbesondere Derivate des Benzoylmethans in Frage. Die UV-A und UV-B-Filter können selbstverständlich auch in Mischungen eingesetzt werden, z.B. Kombinationen aus den Derivaten des Benzoylmethans, z. B. 4-tert.-Butyl-4'-methoxydibenzoylmethan (Parsol® 1789) und 2-Cyano-3,3-phenylzimtsäure-2-ethyl-hexylester (Octocrylene) sowie Estem der Zimtsäure, vorzugsweise 4-Methoxyzimtsäure-2-ethylhexylester und/oder 4-Methoxyzimtsäurepropylester und/oder 4-Methoxyzimtsäureisoamylester. Häufig werden derartige Kombinationen mit wasserlöslichen Filtern wie z. B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen auch unlösliche Lichtschutzpigmente, nämlich feindisperse Metalloxide bzw. Salze in Frage. Beispiele für geeignete Metalloxide sind insbesondere Zinkoxid und Titandioxid. Als Salze können Silicate (Talk), Bariumsulfat oder Zinkstearat eingesetzt werden. Die Oxide und Salze werden in Form der Pigmente für hautpflegende und hautschützende Emulsionen verwendet.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt.

### Biogene Wirkstoffe

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambaranussextrakt und Vitamin komplexe zu verstehen.

### Desodorierende Wirkstoffe

Desodorierende Wirkstoffe wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend eignen sich als deosodorierende Wirkstoffe u.a. keimhemmende Mittel, Enzyminhibitoren, Geruchsabsorber oder Geruchsüberdecker.

### Anitranspirante Wirkstoffe

Antitranspirant-Wirkstoffe reduzieren durch Beeinflussung der Aktivität der ekkrinen Schweißdrüsen die Schweißbildung, und wirken somit Achselnässe und Körpergeruch entgegen. Als adstringierende Antitranspirant-Wirkstoffe eignen sich vor allem Salze des Aluminiums, Zirkoniums oder des Zinks.

Solche geeigneten antihydrotisch wirksamen Wirkstoffe sind z. B. Aluminiumchlorid, Aluminiumchlorhydrat, Aluminiumdichlorhydrat, Aluminiumsesquichlorhydrat und deren Komplexverbindungen z. B. mit Propylenglycol-1,2, Aluminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin.

### Insekten-Repellentien

Als Insekten-Repellentien kommen beispielsweise N,N-Diethyl-m-toluamid, 1,2-Pentandiol oder 3-(N-n-Butyl-N-acetyl-amino)-propionsäureethylester), welches unter der Bezeichnung Insect Repellent® 3535 von der Merck KGaA vertrieben wird, sowie Butylacetylaminopropionate in Frage.

### Selbstbräuner und Depigmentierungsmittel

Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Iso-propylalkohol, oder Polyole eingesetzt werden. Polyole, die hier in Betracht kommen, besitzen vorzugsweise 2 bis 15 Kohlenstoffatome und mindestens zwei Hydroxylgruppen.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen, Harzen und Balsamen. Weiterhin kommen tierische Rohstoffe, wie beispielsweise Zibet und Castoreum sowie synthetische Riechstoffverbindungen vom Typ der Ester, Ether, Aldehyde, Ketone, Alkohole und Kohlenwasserstoffe in Frage.

### Farbstoffe

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Diese Farbstoffe werden üblicherweise in Konzentrationen von 0,001 bis 0,1 Gew.-%, bezogen auf die gesamte Mischung, eingesetzt.

### Beispiele

**Tabelle 1**

| | **Erfindungsgemäße Zusammensetzung** | | **Absorption** | **Öligkeit** | **Weichheit** | **Samtigkeits-Faktor** |
|---|---|---|---|---|---|---|
| **1** | 50% Di-n-octylcarbonat | 50% Diethyldodecan | 2 | 2 | 1 | 1 |
| **2** | 30% Di-n-octylcarbonat | 70% Diethyldodecan | 1 | 1 | 1 | 1 |
| **3** | 10% Di-n-octylcarbonat | 90% Diethyldodecan | 1 | 1 | 1 | 1 |
| **4** | 50% Di-2-ethylhexylcarbonat | 50% Diethyldodecan | 2 | 2 | 1 | 1 |
| **5** | 50% Di-n-octylcarbonat | 50% Isohexadecan | 1 | 2 | 1 | 1 |
| **6** | 50% Di-n-octylcarbonat | 50% Didecen | 1 | 1 | 1 | 1 |
| **7** | 90% Di-n-octylcarbonat | 10% Diethyldodecan | 2 | 2 | 2 | 2 |
| **V1** | 100% Cyclopentasiloxan | | 1 | 1 | 2 | 1 |
| **V2** | 100% Di-n-octylcarbonat | | 4 | 3 | 4 | 3 |
| **V3** | 100% Diethyldodecen | | 3 | 4 | 3 | 3 |
| **V4** | 100% Didecen | | 3 | 4 | 4 | 4 |
| **V5** | 50% Di-n-octylcarbonat | 50% Di-n-octylether | 2 | 3 | 2 | 2 |
| **V6** | 50% Cocoglyceride | 50% Didecen | 4 | 4 | 4 | 4 |
| **V7** | 50% Di-n-octylcarbonat | 50% Cocoglyceride | 5 | 4 | 4 | 4 |
| **V8** | 50% Dibutyladipat | 50% Di-n-octylcarbonat | 3 | 4 | 5 | 5 |
| **V9** | 50% Dibutyladipat | 50% Diethyldodecan | 3 | 4 | 3 | 3 |

Bewertung im Vergleich zu einem Cyclomethicone (Dow Corning 245)

1 = exzellent; 2 = sehr gut; 3 = gut, 4 = mittel; 5 = unbefriedigend

Sensorisch am besten werden Mischungen aus Di-n-octylcarbonat und Diethyldodecane bzw. Didecen bewertet, wobei es sich bei der Alkan-Komponente um ein Isomerengemisch handelt.

Testgruppe: 10 erfahrene und geschulte Freiwillige 10µl der o.g. Zusammensetzungen wurden mittels einer Mikropipette auf die unbehaarte Seite der Unterarme der Probanden appliziert und mit den Fingern der Hände der kontralateralen Seite eingerieben. Die Beurteilung der Sensorik erfolgte während und nach der Absorption.

Der Sensorik-Test wurde an 10 Probanden durchgeführt, wie in dem Buch "Cosmetic Lipids and the Skin Barrier" (Marcel Dekker Verlag New York, 2002, Ed. Thomas Förster, S. 319-352) beschrieben.

## Patentansprüche

1. Bei 20°C und Normaldruck flüssige, wasserfreie Zusammensetzung auf Ölbasis, enthaltend
(a) 2 - 90 Gew.-% wenigstens eines linearen und/oder verzweigten Dialkylcarbonats,
(b) 2 - 95 Gew.-% wenigstens eines linearen und/oder verzweigten, gesättigten oder ungesättigten Alkans mit 8 bis 40 Kohlenstoffatomen.

2. Zusammensetzung gemäß wenigstens einem der Ansprüche 1 oder 2, **dadurch gekennzeichnet, dass**
(a) wenigstens 10 Gew.-% eines linearen und/oder verzweigten Dialkylcarbonats und
(b) wenigstens 50 Gew.-% eines linearen und/oder verzweigten, gesättigten oder ungesättigten Alkans mit 8 bis 40 Kohlenstoffatomen
enthalten sind.

3. Zusammensetzung gemäß wenigstens einem der Ansprüche 1 bis 2, **dadurch gekennzeichnet, dass** keine zusätzlichen Öle oder Wachse enthalten sind.

4. Zusammensetzung gemäß wenigstens einem der Ansprüche 1 bis 3, **dadurch gekennzeichnet, dass** das Alkan 10 bis 30 vorzugsweise 12 bis 20, und besonders bevorzugt 16 bis 20 Kohlenstoffatome aufweist.

5. Zusammensetzung gemäß wenigstens einem der Ansprüche 1 bis 4, **dadurch gekennzeichnet, dass** das Alkan (b) ein nicht-cyclisches Alkan oder ein Gemisch aus nicht-cyclischen Alkanen ist.

6. Zusammensetzung gemäß wenigstens einem der Ansprüche 1 bis 5, **dadurch gekennzeichnet, dass** das Alkan (b) ein Gemisch aus Alkanen ist, welches wenigstens 10 Gew.-% verzweigte Alkane bezogen auf die Gesamtmenge der Alkane enthält.

7. Zusammensetzung gemäß wenigstens einem der Ansprüche 1 bis 6, **dadurch gekennzeichnet, dass** das Alkan (b) eine Gemisch aus Alkanen ist, welches mehr als 1 Gew.-% 5,8-Diethyldodecan und/oder mehr als 1 Gew.-% Didecen enthält.

8. Zusammensetzung gemäß wenigstens einem der Ansprüche 1 bis 7, **dadurch gekennzeichnet, dass** das Dialkylcarbonat Alkylketten mit 6 bis 24 Kohlenstoffen aufweist.

9. Zusammensetzung gemäß wenigstens einem der Ansprüche 1 bis 8, **dadurch gekennzeichnet, dass** das Dialkylcarbonat (a) Di-n-octylcarbonat oder Di-(2-ethylhexyl)carbonat oder ein Gemisch dieser Substanzen ist.

10. Zusammensetzung gemäß wenigstens einem der Ansprüche 1 bis 9, **dadurch gekennzeichnet, dass**
(a) 2 - 90 Gew.-% Di-n-octylcarbonat und
(b) 2 - 95 Gew.-% eines Diethyldodecans oder eines Didecens oder eines beliebigen Isomerengemisches dieser Substanzen
enthalten ist.

11. Kosmetische Mittel enthaltend eine bei 20°C und Normaldruck flüssige, wasserfreie Zusammensetzung auf Ölbasls, enthaltend
(a) 2 - 90 Gew.-% wenigstens eines linearen und/oder verzweigten Dialkylcarbonats,
(b) 2 - 95 Gew.-% wenigstens eines linearen und/oder verzweigten, gesättigten oder ungesättigten Alkans mit 10 bis 20 Kohlenstoffatomen mit der Maßgabe, dass keine zusätzlichen Öle oder Wachse enthalten sind.

12. Kosmetisches Mittel enthaltend 0,1.- 50 Gew-% einer Zusammensetzung gemäß Anspruch 11, **dadurch gekennzeichnet, dass** sie frei von Silikonölen ist.

13. Verwendung einer Zusammensetzung gemäß einem der Ansprüche 1 bis 10 als Silikonersatz in kosmetischen und/oder pharmazeutischen Zubereitungen.

## Claims

1. An anhydrous oil-based composition that is liquid at 20°C and atmospheric pressure, comprising
(a) 2-90% by weight of at least one linear and/or branched dialkyl carbonate,
(b) 2-95% by weight of at least one linear and/or branched, saturated or unsaturated alkane having 8 to 40 carbon atoms.

2. The composition according to claim 1, wherein
(a) at least 10% by weight of a linear and/or branched dialkyl carbonate and
(b) at least 50% by weight of a linear and/or branched, saturated or unsaturated alkane having 8 to 40 carbon atoms
are present.

3. The composition according to at least one of claims 1 to 2, wherein no additional oils or waxes are present.

4. The composition according to at least one of claims 1 to 3, wherein the alkane has 10 to 30, preferably 12 to 20, and particularly preferably 16 to 20, carbon atoms.

5. The composition according to at least one of claims 1 to 4, wherein the alkane (b) is a noncyclic alkane or a mixture of noncyclic alkanes.

6. The composition according to at least one of claims 1 to 5, wherein the alkane (b) is a mixture of alkanes which comprises at least 10% by weight of branched alkanes, based on the total amount of alkanes.

7. The composition according to at least one of claims 1 to 6, wherein the alkane (b) is a mixture of alkanes which comprises more than 1% by weight of 5,8-diethyldodecane and/or more than 1% by weight of didecene.

8. The composition according to at least one of claims 1 to 7, wherein the dialkyl carbonate has alkyl chains with 6 to 24 carbons.

9. The composition according to at least one of claims 1 to 8, wherein the dialkyl carbonate (a) is di-n-octyl carbonate or di(2-ethylhexyl) carbonate or a mixture of these substances.

10. The composition according to at least one of claims 1 to 9, wherein
(a) 2-90% by weight of di-n-octyl carbonate and
(b) 2-95% by weight of a diethyldodecane or a didecene or any desired isomer mixture of these substances
are present.

11. A cosmetic composition comprising an anhydrous oil-based composition that is liquid at 20°C and atmospheric pressure, comprising
(a) 2-90% by weight of at least one linear and/or branched dialkyl carbonate,
(b) 2-95% by weight of at least one linear and/or branched, saturated or unsaturated alkane having 10 to 20 carbon atoms with the proviso that no additional oils or waxes are present.

12. A cosmetic composition comprising 0.1-50% by weight of a composition according to claim 11, wherein it is free from silicone oils.

13. The use of a composition according to any one of claims 1 to 10 as silicone substitute in cosmetic and/or pharmaceutical preparations.

## Revendications

1. Composition anhydre à base huileuse, liquide à 20 °C et sous la pression normale, contenant
(a) 2 - 90 % en poids d'au moins un carbonate de dialkyle linéaire et/ou ramifié,
(b) 2 - 95 % en poids d'au moins un alcane linéaire et/ou ramifié, saturé ou insaturé, ayant de 8 à 40 atomes de carbone.

2. Composition selon la revendication 1, **caractérisée en ce que** sont contenus
(a) au moins 10 % en poids d'un carbonate de dialkyle linéaire et/ou ramifié et
(b) au moins 50 % en poids d'un alcane linéaire et/ou ramifié, saturé ou insaturé, ayant de 8 à 40 atomes de carbone.

3. Composition selon au moins l'une quelconque des revendications 1 et 2, **caractérisée en ce que** des huiles ou cires supplémentaires ne sont pas contenues.

4. Composition selon au moins l'une quelconque des revendications 1 à 3, **caractérisée en ce que** l'alcane a de 10 à 30, de préférence de 12 à 20, et de façon particulièrement préférée de 16 à 20 atomes de carbone.

5. Composition selon au moins l'une quelconque des revendications 1 à 4, **caractérisée en ce que** l'alcane (b) est un alcane acyclique ou un mélange d'alcanes acycliques.

6. Composition selon au moins l'une quelconque des revendications 1 à 5, **caractérisée en ce que** l'alcane (b) est un mélange d'alcanes qui contient au moins 10 % en poids d'alcanes ramifiés, par rapport à la quantité totale des alcanes.

7. Composition selon au moins l'une quelconque des revendications 1 à 6, **caractérisée en ce que** l'alcane (b) est un mélange d'alcanes qui contient plus de 1 % en poids de 5,8-diéthyldodécane et/ou plus de 1 % en poids de didécène.

8. Composition selon au moins l'une quelconque des revendications 1 à 7, **caractérisée en ce que** le carbonate de dialkyle comporte des chaînes alkyle ayant de 6 à 24 atomes de carbone.

9. Composition selon au moins l'une quelconque des revendications 1 à 8, **caractérisée en ce que** le carbonate de dialkyle (a) est le carbonate de di-n-octyle ou le carbonate de di-(2-éthylhexyle) ou un mélange de ces substances.

10. Composition selon au moins l'une quelconque des revendications 1 à 9, **caractérisée en ce que** sont contenus
(a) 2 - 90 % en poids de carbonate de di-n-octyle et
(b) 2 - 95 % d'un diéthyldodécane ou d'un didécène ou d'un mélange quelconque d'isomères de ces substances.

11. Produit cosmétique contenant une composition anhydre à base huileuse, liquide à 20 °C et sous la pression normale, contenant
(a) 2 - 90 % en poids d'au moins un carbonate de dialkyle linéaire et/ou ramifié,
(b) 2 - 95 % en poids d'au moins un alcane linéaire et/ou ramifié, saturé ou insaturé, ayant de 10 à 20 atomes de carbone, étant entendu que des huiles ou cires supplémentaires ne sont pas contenues.

12. Produit cosmétique contenant 0,1 - 50 % en poids d'une composition selon la revendication 11, **caractérisé en ce qu'**il est exempt d'huiles de silicone.

13. Utilisation d'une composition selon l'une quelconque des revendications 1 à 10, en tant que substitut de silicone dans des préparations cosmétiques et/ou pharmaceutiques.
